# EUROPÄISCHE PATENTANMELDUNG

(11) **EP 1 136 092 A2**
(43) Veröffentlichungstag der Anmeldung: **26.09.2001**
(21) Anmeldenummer: 01105986.2
(22) Anmeldetag: 10.03.2001
(51) Int. Cl.: A61M 5/44

(54) **Verfahren und Einrichtung zur Aufwärmung von Infusionsflüssigkeiten zur komfortablen Verabreichung bei Dauerinfusionen an Menschen und Tieren**

(30) Priorität: 17.03.2000 DE 10013456
(71) Anmelder: Grieshaber, Wilhelm, 67071 Ludwigshafen /Rh. (DE)
(72) Erfinder: Grieshaber, Wilhelm, 67071 Ludwigshafen /Rh. (DE)

(57) **Zusammenfassung**

Verfahren und Einrichtung zur komfortablen Anwärmung von Infusion.-, Transfusion.- und Dialyseflüssigkeiten, welche mit trockener Wärme - bei Nutzung herkömmlicher Infusionsbestecke - Wärmeenergie auf in den Übertragungsschläuchen geführten Medien übertragen, versehen mit selbstregelnder Temperaturüberwachung und Sicherheitsabschaltung. Die Medien werden erst kurz vor dem Eintritt in den Infusion-/Transfusion-Empfänger erwärmt.

Die bei kumulativer Vorerwärmung gefürchteten Überwärmungen zum Ausgleich der nachträglichen Abkühlung die mögliche Zersetzung des Mediums bei Dauerwarmhaltung sowie die Bildung von Wärmenester und Verunreinigungen durch Risse, Diffiusion etc. sind ausgeschlossen.

Die vom Empfänger als komfortabel empfundene Verabreichungstemperatur entlastet dessen Wärmehaushalt und läßt seinen Körper unbelasteter und schneller die gewünschte Wirkung der Infusion erreichen.

## Beschreibung

Bisher beschränkt sich im human- und veterinärmedizinischen Bereich die Kontrolle bei der Verabreichung von Infusionen, Transfusionen und Dialyseflüssigkeiten in Bezug auf die temperaturmäßige Handhabung nur auf die allgemeinen Lagertemperaturen. Vor Verabreichung wird zur Homogenisierung der Medien durch Schütteln oder dergl. das besondere Augenmerk auf Viskosität des Mediums gerichtet. Ein all zu großer Kontrast zur Körpertemperatur des Empfängers bleibt nahezu unberücksichtigt.

Die Medien sind mit wenigen Ausnahmen zur freien Lagerung in gedunkelten Räumen bei Zimmertemperaturen geeignet.

Unterstellt man ca. 18 - 20 ° C als mögliche Lagertemperatur, so ist sie ungleich niedrig gemessen an der in engen Grenzen durch den menschlichen Körper geregelten Körpertemperatur von ca. 36 ° C. Entsprechendes gilt für den Tierbereich.

Alleine in den Operations-, Wach- und den Flüssigkeitsaustauschstationen in Krankenhäusern sind Wärmegeräte für Transfusions-Medien bekannt und finden dort unter intensivmedizinischen Bedingungen ihren Einsatz.
Geräte der dort angewandten Art sind unter ständiger Aufsicht des Fachpersonales und bedingen in der Regel spezielle, Manipulationen, aufwendige anlagenbedingte Sonderbehältnisse, Umfüllvorgänge, besondere Wärmetauscher, auch Wasserbäder und speziell dazu passende Verbindungselemente und Schläuche.
Auf den Behandlungsstationen in Krankenhäusern und in der ambulanten Krankenversorgung werden bisher Möglichkeiten des Temperatur-Angleiches der Medien an die Körpertemperatur des Empfängers nicht praktiziert.

Infusionen z.B. werden in der Regel nur verabreicht, um dem Organismus eines Körpers über dessen Gefahrenmomente, Schwächezustände oder dergl. hinwegzuhelfen. Somit werden gerade bei hohen Organismus- und Kreislaufbelastungen durch Dareichung in Bezug auf die Körpertemperatur zu niedriger Infusionstemperatur zusätzliche Belastungen hervorgerufen.
Die z.B. in der Intensivmedizin verwendeten Katheder führen die verabreichten Flüssigkeiten oft punktuell an besonders unterkühlungsempfindliche Körperregionen, wie z. B. in die Hohlvene am Kehlkopf- und Stimmbandbereich, wo sich bei weiterer Nutzung der gelegten Zuführungen im stationären Bereich, insbesondere bei Dauerinfusionen, Irritationsschmerzen einstellen bei zu geringer Dareichungstemperatur.
Insgesamt wird der Körper zur Erbringung einer größeren Wärmeleistung zum Ausgleich seiner Temperatur herausgefordert, gerade zu Zeiten seiner schwächsten allgemeinen Verfassung.

Es ist bekannt, daß eine Heranführung durch Wärmung der Infusionsflüssigkeit bis möglichst nahe an die Körpertemperatur des Empfängers, insbesondere bei großen Infusionsmengen und oder länger andauernder Verabreichung, besondere Vorteile bringt:
- Keine zusätzliche Belastung des Organismus zwecks Erbringung einer Wärmeleistung.
- Schnellere und umfassende Reaktion des Organismus auf die Infusion.
- Vermeidung von Nebenwirkungen durch lokale Unterkühlungen.
- Besseres, allgemeines und positives Empfinden des Empfängers auf die Wärme der Flüssigkeit.

Es wurde gefunden, daß man unter Beibehaltung der üblicherweise benutzten Infusionsbestecke und Einlegen des Zuführungsschlauches zwischen der Tropfregulierung und dem Anschluß des Schlauches an die Einführungskanüle des Empfängers in eine Wärmeinrichtung die vorgenannten Positivpunkte, die bei Erwärmung des Mediums auf möglichst Körpertemperatur entstehen, zusammenführen kann.

An einem gut wärmeleitenden Körper (1) wird auf der einen Seite z.B. eine durch elektrische Energie versorgte Heizung (2) angebracht und diese nach der Rückseite (3) soweit abisoliert, damit möglichst wenig Abstrahlung der Heizleistung nach der freien Seite verloren geht und möglichst die gesamte Wärmeleistung in dem Körper sich gleichmäßig ausbreitet.
Auf der Gegenseite der Heizeinrichtung ist eine Nut (4) in den beheizten Körper eingelassen, die es erlaubt, einen üblicherweise bekannten Transfusionsschlauch einzulegen und über/durch den beheizten Körper durchzuführen.

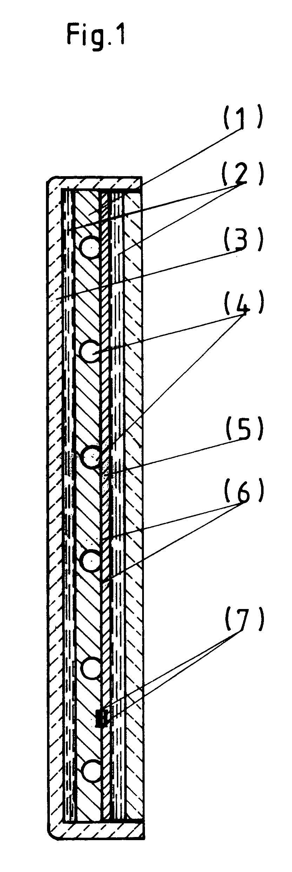

Eine der möglichen Ausführungsformen hierzu ist eine Platte (Fig. 1). Die Nut zur Aufnahme des Schlauches ist so ausgebildet, daß sie dem Durchmesser und normalen Form-Verhalten des Schlauches möglichst nahe kommt. Bewußt wird die Tiefe der Nut - gemessen von der Oberfläche des Körpers - um wenige Zehntel des Schlauchdurchmessers kleiner gehalten, damit der Schlauch bei üblichem losem Einlegen geringfügigen Überstand über die Oberfläche aufweist. Eine Abdeckung (5) über die exakt geplante Oberfläche (6) des Körpers erreicht bei Andrücken auf den Körper zweierlei:
- Sie drückt den Schlauch mit einer Vorspannung in die Konturen der aus dem Körper ausgehobenen Nut in diesen so fest an, daß ein intensiverer Wärmeübergang als bei losem Einlegen erfolgt.
- Über die freien planen Kontaktflächen zwischen den Nuten erwärmt sich die Abdeckung gleichermaßen wie der beheizte Körper selbst und bildet damit gleichzeitig eine zusätzliche erwärmte Kontaktfläche, die Andruckfläche für den Schlauch.

Eine mögliche zusätzliche Beheizung der Abdeckung ähnlich der Beheizung der Grundkörpers gewährleistet mit größerer Sicherheit, daß nicht nur Teilgrößen der Temperatur des Wärmekörpers mittels der Abdeckung übertragen werden, sondern eine überwachte Maximaltemperaturübertragung gleich der des Wärmkörpers selbst effektiv auch an den Berührungsflächen der Abdeckung mit der Schlauchperipherie eintritt.

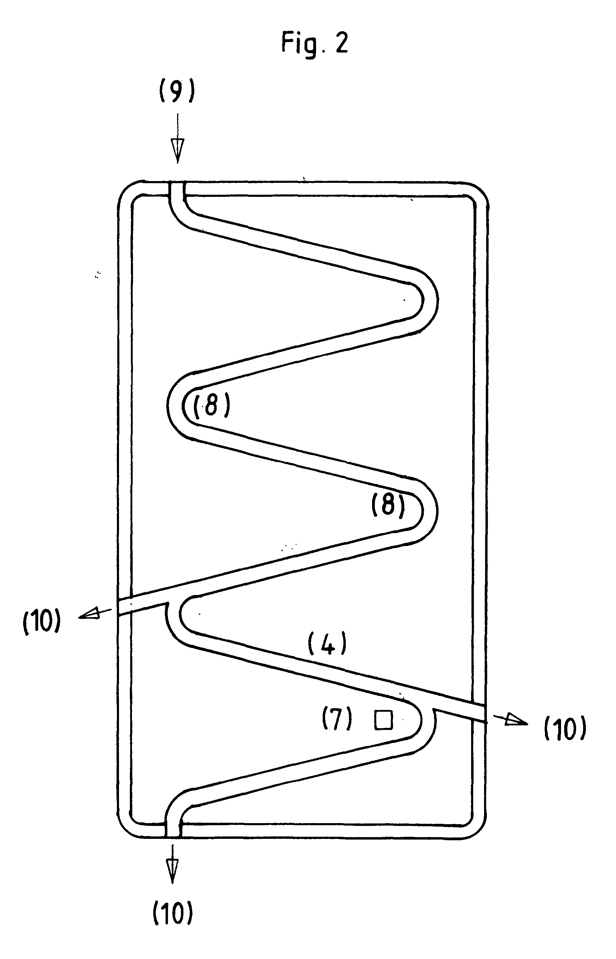

Die Nut - das Schlauchbett (4) - ist auf dem beheizten Körper (1) in meanderartigen Schlingen so ausgebildet, daß zwecks besserer Durchmischung des Mediums in dem Schlauch die Nut mindestens in einer Ebene gegenläufige Kurvenausprägungen (8) erfährt. (Fig. 2)

Sowohl der Wärmkörper als auch die Abdeckung mit oder ohne zusätzliche Energiezuführung können auch aus einem plastisch verformbaren Material teilweise z.B. in einer Schicht oder auch ganz bestehen, sofern sie dazu geeignet sind, gute Wärmeüberträger zu sein und eine optimale Berührungsfläche/Ummantelung mit der Schlauchform erzeugt werden kann.

Die Wärmeeinspeisung auf den Wärmkörper ist mit allen bekannten Möglichkeiten der Wärmeübertragung zu realisieren. Z. B. elektrische Beheizung, gebündelte Lichtstrahlung, Strahlungswärme aus anderen warmen Umgebungen unter Einschluß auch einer offenen Flamme wie einer Kerze.

In der bevorzugten Ausführungsart besteht die Wärmequelle aus einem aus Sicherheitsgründen mit Kleinstspannung versorgten Heizelement, dessen Material die Eigenschaft hat bei steigender Temperatur seinen elektrischen Widerstand ebenfalls zu erhöhen. Diese Art des Einsatzes einer Regelung "PTC" (positiver Temperaturkoeffizient) gewährleistet das selbsttätige Nachregeln der Heizleistung je nach der gewünschten Temperatur in Abhängigkeit von der Abkühlung der Wärmeinrichtung sei es durch Wärmeabgabe an den Infusionsschlauch und das Medium in ihm oder auch Abstrahlung an das Umfeld. Es wird somit auch unterschiedlichen Durchlaufmengen bei gleichbleibender Temperatur Rechnung getragen.

Ein zusätzlich im Kontaktbereich des Schlauches angeordneter Thermostat (7) dient der sicheren Abschaltung der Energiezufuhr bei eventuellem Überschreiten der maximal zugelassenen Übertragungstemperatur.

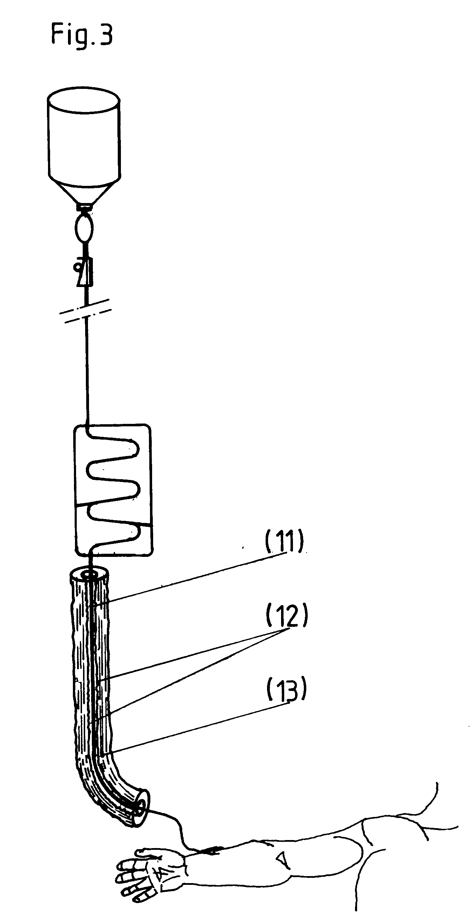

Die hier beschriebene Einrichtung ermöglicht eine komfortablere Verabreichung von Infusionsmedien zu gewährleisten, ohne daß die herkömmlich bekannten Infusionsbestecke ausgetauscht oder verändert werden müssen. Alleine der Wärmeübergangswert von dem maximal auf 36°C erwärmten Wärmkörper und seiner eventuellen Abdeckung über die Schlauchwandung auf das Medium im Schlauch ist ausschlaggebend für den in der Zeit des Mediendurchflusses im beheizten Schlauchbereich übertragbaren Wärmezugang. Erfahrungsgemäß ist die Temperaturzunahme direkt abhängig von der Länge des beheizten Schlauchanteiles, durch den das Medium fließt und somit der Verweildauer im Wärmbereich. Eine wesentliche Erhöhung des Aufheizungswertes ist auch dadurch gegeben, daß die nach der Wärmeinrichtung, beispielsweise zum Empfänger führende Schlauchleitung, möglichst kurz gehalten wird, um die nachträgliche Wiederabkühlung auf Umgebungstemperatur zu minimieren, bzw. der nachgeschaltete Schlauchbereich (11) mit einer Wärmeisolierung (12) versehen wird, die zusätzlich mit einer wärmeverlustreduzierenden Begleitheizung (13) ausgebildet sein kann. (Fig. 3)

Je nach Ausgangstemperatur des Infusionsmediums und der gewünschten Eintrittstemperatur des angewärmten Mediums beim Empfänger ist es sinnvoll, Einrichtungen der vorbeschriebenen Art einzusetzen, die in der beschriebenen Schlauchnut so ausgebildet sind, daß die Nut einen Eingang (9) in den Wärmkörper, aber mehrfach unterschiedliche Ausgänge (10) aufweist, die unterschiedliche beheizte Schlauchlängen als Aufheizstrecken bei konstanter Wärm-temperatur ermöglichen (Fig. 2) In jedem Falle kann so über die zur Wärmung eingespannte Schlauchlänge die Abgabetemperatur vorbestimmt werden bis hin zur Maximaltemperatur von ca. 36° C.

Die Nut ist vorzugsweise so angeordnet, daß sie zum Eintritt des mediumführenden Schlauches hin ständig ansteigt und somit eventuelle Blasenbildungen im Wärmbereich zur Be- und Entlüftungseinrichtung des Durchflußreglers aufsteigen können.

Bei extremen notwendigen Temperaturerhöhungen können auch mehrere der vorbeschriebenen Wärmeinrichtungen auf die Länge des Schlauches eingefügt werden, da jede der Einrichtungen von sich aus in sich abgeschlossen regelt und steuert und die Maximaltemperatur niemals über die verträglichen 36° C hinausgefahren werden kann.

Bei der vorbeschriebenen Einrichtung und ihrer möglichen Handhabungen wird der besondere Vorteil darin gesehen, daß es damit möglich ist, die Vorteile der auf Körpertemperatur herangeführten Infusionsmedien zu erzielen. Die Medien werden jedoch erst zum Zeitpunkt kurz vor Eintritt in den Empfängerkörper angewärmt. Es werden somit keine eventuell mögliche Zersetzungs- oder Umsetzungsprodukte sich einstellen, wie sie beispielsweise eintreten können, wenn die gesamte Dareichungsmenge in ihrem Transportbehältnis bereits vorher extern z.B. in einem Wasserbad aufgewärmt oder auch im Hinblick auf nachträgliche Abkühlung überwärmt worden ist. In jedem Falle besteht bei der hier beschriebenen trockenen Erwärmung der Medien nicht die gefahrvolle Möglichkeit der Bildung von Diffusion, Mikrorissen, somit evtl. Verunreinigungen der Medien. Die Nachteile kumulativ erwärmter Medien, die sich während der Verabreichungszeit ohnedies und beim Durchfluß durch die dünne Zuleitung zusätzlich auf die Umgebungstemperatur zurückkühlen, sind ausgeschlossen.

Die bei der Erwärmung durch Mikrowellen- oder auch Infrarotbestrahlung möglichen gefürchteten Wärmenester können nicht eintreten.

## Patentansprüche

1. Verfahren und Einrichtung zur komfortablen Verabreichung von Infusions-, Transfusions- und Dialyse-Flüssigkeiten bei Menschen und Tieren durch Anwärmung **dadurch gekennzeichnet, daß** herkömmliche bekannte Verbindungsteile und Schläuche ohne zusätzliche spezielle Sonderteile die Flüssigkeiten führen und in diesen durch trockene Hitze, die sie umgibt, die Flüssigkeit in den Zuführungsverbindungen zum Körper, kurz vor dem Eintritt in diesen, erwärmt.

2. Verfahren und Einrichtung nach 1 **dadurch gekennzeichnet, daß** einem Körper bestehend aus gut wärmeübertragendem Material Wärme zugeführt wird durch elektrische Energie.

3. Verfahren und Einrichtung nach 1-2 **dadurch gekennzeichnet, daß** die Energiezufuhr durch Strahlung einer Wärmequelle zugeleitet wird.

4. Verfahren und Einrichtung nach 1 - 3 **dadurch gekennzeichnet, daß** der Körper mit mindestens einer Nut versehen ist, in die der das Medium führende Zuführungsschlauch eingelegt ist.

5. Verfahren und Einrichtung nach 1 - 4 **dadurch gekennzeichnet, daß** die Nut so ausgebildet ist, daß sie die Außenform des Zuführungsschlauches möglichst exakt wiederspiegelt.

6. Verfahren und Einrichtung nach 1 - 5 **dadurch gekennzeichnet, daß** die Nut eine geringfügig geringere Höhe aufweist, als das Außenmaß des Schlauches.

7. Verfahren und Einrichtung gemäß 1 - 6 **dadurch gekennzeichnet, daß** eine Abdeckung über die Fläche des Körpers passend ist und sie den Schlauch mit Vorspannung in der gesamten Fläche der Nut fest anpreßt.

8. Verfahren und Einrichtung gemäß 1 - 7 **dadurch gekennzeichnet, daß** auch die Abdeckung Wärme aus einer eigenen Wärmequelle erhält.

9. Verfahren und Einrichtung gemäß 1 - 8 **dadurch gekennzeichnet, daß** der Wärmkörper und die Abdeckung plastisch verformbar sind.

10. Verfahren und Einrichtung gemäß 1 - 9 **dadurch gekennzeichnet, daß** die Nut in ihrer Länge in mindestens zwei gegenläufigen Ebenen angeordnet ist.

11. Verfahren und Einrichtung gemäß 1 - 10 **dadurch gekennzeichnet, daß** die Nut längere oder kürzere Einlegelängen zuläßt.

12. Verfahren und Einrichtung gemäß 1 - 11 **dadurch gekennzeichnet, daß** die Nut mit zum Eintritt des Mediums ständig ansteigendem Verlauf eingearbeitet ist.

13. Verfahren und Einrichtung gemäß 1 -12 **dadurch gekennzeichnet, daß** eine Heizeinrichtung je nach Temperatur ihren Widerstand verändert und eine Konstanttemperatur selbsttätig regelt.

14. Verfahren und Einrichtung gemäß 1 - 13 **dadurch gekennzeichnet, daß** eine Sicherheitseinrichtung die Überschreitung einer vorgegebenene Temperatur verhindert.

15. Verfahren und Einrichtung gemäß 1 - 14 **dadurch gekennzeichnet, daß** die Wärmeeinrichtung bei elektrischer Versorgung mit Kleinstspannung versorgt wird.

16. Verfahren und Einrichtung gemäß 1 - 15 **dadurch gekennzeichnet, daß** die Wärmeeinrichtung und die dahinter weiterführende Schlauchleitung in einer wärmeabstrahlungsverhindernden Isolation geführt wird.

17. Verfahren und Einrichtung gemäß 1 - 16 **dadurch gekennzeichnet, daß** die Wärmeerhaltungs-lsolation mit einer Unterstützungsheizung versehen ist.
